Europäisches Patentamt

European Patent Office

Office européen des brevets

(11) Publication number: 0 372 702 A1

# EUROPEAN PATENT APPLICATION

(21) Application number: 89310917.3

(22) Date of filing: 24.10.89

(51) Int. Cl.⁵: A61K 35/52, //A61K37/54

(30) Priority: 25.10.88 GB 8824926

(43) Date of publication of application:
13.06.90 Bulletin 90/24

(84) Designated Contracting States:
ES GR

(71) Applicant: THE VICTORIA UNIVERSITY OF
MANCHESTER
Oxford Road
Manchester. M13 9PL(GB)

(72) Inventor: Chantler, Eric Nelson
45 Longhurst Lane Marple Bridge
Stockport Cheshire SK6 5AE(GB)
Inventor: Sharma, Rekha
19 Haw Lane Yeadon
Leeds Yorkshire LS19 7XR(GB)

(74) Representative: Tunnicliffe, Peter Barry
Westfields House 5 Vincent Drive Lache
Lane
Chester, CH4 7RQ(GB)

(54) Gynaecological procedure.

(57) A method for treatment of semen/spermatozoa to enhance their ability to fertilise ova, using a reagent solution containing a protease (especially chymotrypsin) and a saccharide (especially D-galactose) to remove antagonistic antibodies and protect the spermatozoa. The treated spermatozoa are freed from extraneous materials by washing and concentrated by centrifugation, and may then be used in assisted conception techniques. They may be preserved by conventional means. Also provided is a convenient form of "kit" for easy clinical preparation of the reagent solution.

EP 0 372 702 A1

EP 0 372 702 A1

## Gynaecological Procedure.

This invention relates to an improved gynaecological procedure, and more particularly to a procedure for treating spermatozoa to increase their ability to fertilise ova and to reduce/overcome the problems of infertility resulting from the presence of anti-sperm antibodies in the semen.

It is known that spermatozoa can be affected adversely by various antagonistic materials -- antibodies and other materials -- which are associated with infertility problems. These materials usually reduce motility but also reduce the ability of spermatozoa to fertilise ova by changes not associated with motility. The mechanism for this is not entirely understood, but is believed to be associated with antibodies which are presented to, and bind with, the spermatozoa on ejaculation.

Hitherto, no clinically applicable method, compatible with the retention of fertilising capability, has been found for displacing these antagonistic materials from spermatozoa in ejaculated semen.

We have now found that this problem can be overcome in a high proportion of cases and the effect of the antagonistic material can be reduced or modified in a manner which is not antagonistic to fertilisation.

Thus according to our invention we provide a method for treating semen which comprises

(a) mixing the semen with a reagent solution containing a protease and a saccharide,

(b) incubating the mixture under conditions at which the protease is active, and then

(c) separating the spermatozoa from the resulting mixture.

The invention also provides reagents (especially reagent solutions) useful for the treatment of semen for use in the above method, which comprise a solution of a saccharide and a protease, and also preparations containing spermatozoa and having reduced antibody content, produced by the above method.

The function of the protease is not entirely understood, but it is believed that it acts in part to hydrolyse proteinaceous material (proteins and/or glycoproteins) and partly to have a liquefying effect on the semen.

The protease may be any protease (proteolytic enzyme) which is free from DNAase (i.e. enzyme which degrades DNA) and should be active at a pH which is not unduly harmful or antagonistic towards spermatozoa. Preferably, therefore it should be active at substantially neutral pH, or very slightly on the acid side of strictly neutral, so as to preserve the spermatozoa and to reduce the severity of enzymic degradation.

Thus it may be any such protease but is preferably a serine protease, for example chymotrypsin. In general, chymotrypsin is preferred because it has the advantage of being most readily obtainable in a sufficiently pure form suitable for human use (i.e. in a sterile form) and because it increases the rate of liquefaction of the semen. As an alternative to a serine protease, a pronase may be used if desired, but at present it is not easily available in commerce and cannot be obtained easily in a sufficiently pure state for use in this invention.

·The saccharide may be any compound containing a structure derived from a carbohydrate (sugar) and conveniently referred to as an "analogue" of it, but is preferably one derived from the sugar galactose. Thus it may be a monosaccharide, or an oligosaccharide (especially one with a galactose entity as a terminal unit. Most conveniently it may be galactose itself. The galactose moiety is preferably that of D-galactose, though,. if desired, those of other enantiomers or mixtures of enantiomers may be used if desired (e.g. D- and/or L-galactose). Alternatives to the sugars themselves include compounds derived from the sugar itself, for example N-acetyl-D-galactosamine.

The saccharide, and especially D-galactose, is a highly advantageous additive, as this appears to act in conjunction with the protease to give an enhanced removal of the antagonistic materials. A convenient proportion is in the range 1.5 to 2.0 grams per 100 ml. of the solution to be mixed with the ejaculate. The co-incubation with the saccharide displaces or inhibits further binding of the antibody, as detected by anti-IgG and anti-IgA binding marking.

The reagent solution for use in the mixing/treatment stage (a) herein may be made in any aqueous medium and most conveniently may contain an amount of the saccharide which gives a concentration of the saccharide in the reagent solution in the range 1.5 to 2.0 grams per 100 ml. of the reagent solution. The concentration of the protease it may contain can vary considerably, depending upon such factors as the purity of the protease and the activity of the protease (and, of course, the specific protease or mixture of proteases used); most conveniently this may be in the range 0.01 to 0.1 grams of the purified protease per 100 ml. of the reagent solution in the case of chymotrypsin, but larger or smaller concentrations may be used depending upon the proteolytic activity of the enzyme used and larger amounts may be used if the enzyme is in a less active form.

The reagent solution may also contain any physiologically acceptable additives. Such additives may be for the purposes of countering infection or organic deterioration of the mixture or components in it, or for

2

assisting in maintaining an environment which is not unduly harmful to the spermatozoa, and may be used in any proportions or combinations which do not have an adverse effect on the spermatozoa. These additives may be included to maintain the solution isotonic (or substantially/nearly so) with body fluids, or may be an antiseptic or antibacterial agent. The reagent solution is preferably substantially neutral or slightly more acid than strictly neutral, for example about one pH unit below the optimum pH at which the enzyme acts. For purposes of storage, the reagent should be kept at a temperature at which the enzyme (protease) is substantially stable, but it is strongly preferred that the reagent solution is freshly made up before use, and is used for the treatment of semen within about an hour, but if possible is used as soon as possible after being made up.

It is important to treat the semen with minimum delay, so it is preferred that the mixing of the semen with the reagent solution for use in the mixing/treatment stage (a) herein should be carried out as speedily as is practicable and with minimum delay after ejaculation -- preferably within 10 seconds, but in less time than this if possible. It is therefore most desirable to mix the semen directly with the reagent solution containing both the components, i.e. the protease and the saccharide.

To secure best results the male donating the semen should preferably ejaculate directly into the reagent solution of the protease and saccharide, which has already been prepared and is at the desired temperature, as this reduces the time factor to the minimum.

The temperature at which the reagent solution is mixed with the semen should be one which does not have a significantly adverse effect on the spermatozoa. It is preferably at or near body temperature. A very suitable temperature is in the range 25 to 30 degrees Centigrade.

After the semen and solution are brought together, further mixing can be carried out by any convenient means which does not impose conditions (for example mechanical forces) on the spermatozoa which might harm them. Most conventional forms of mild mixing may be used, for example simple mixing by inversion of the mixture 5 to 10 times, will suffice. Any high shear or ultrasonic mixing methods should be avoided, as they are lethal to spermatozoa, and even excessive shaking could precipitate proteins by foaming and also damage spermatozoa.

The incubating stage (b) of the method described herein may use any conditions which allow the proteolytic enzyme to hydrolyse protein but not to have any appreciable adverse effect on the spermatozoa themselves. (Similar requirements apply also to the choice of protease itself, as the use of an enzyme which requires a very acid pH to act (e.g. trypsin) can have an unduly adverse effect by virtue of the pH conditions.) These conditions are substantially the conventional standard conditions under which the enzyme is active, but preferably the medium is kept slightly more acid than these, for example about one pH unit below the optimum pH at which the enzyme acts. This slight modification of pH is desirable to preserve the spermatozoa and to reduce the severity of enzymic degradation.

Very convenient conditions are at temperatures in the range of 30 to 37 degrees Centigrade. Higher temperatures would harm the spermatozoa, and lower temperatures would decrease the enzyme activity to an inconveniently low level.

The time required for the treatment (i.e. the incubation, or the total time for which the semen is in contact with the reagent solution containing the protease may also vary, but is conveniently in the range 10 to 20 minutes. to some extent the time and temperature are inter-related but the optimum may be determined by simple trial. In practice the temperature range is limited and long-term exposure to the enzyme is undesirable, so a very convenient time is about 20 minutes.

The amount of the enzyme to be present in the mixture of semen and reagent solution is indicated above, and in the case of chymotrypsin itself may conveniently be approximately 5000 USP units, corresponding to a concentration of the enzyme of about 500 USP units per ml., though larger or smaller amounts and concentrations may be used if desired. Different enzymes tend to be measured in different "units" so this reference to USP units is not appropriate for other enzymes.

The proportion of the reagent (protease/saccharide) solution to the semen may be most conveniently in the range between 1 and 3 parts by volume of the semen for each 10 parts by volume of the solution. Presenting the ratio in this order is more convenient in practice, as the volume of any given sample of semen can be very variable (usually, but not always, about 1 to 3 ml.), and it is convenient to combine this "variable volume" sample with a given volume of reagent solution (e.g. 10 ml.) which is sufficient to treat it.

The main consideration is to dilute out unbound antibody to reduce the binding to spermatozoa. The reduction of the viscosity of the semen also facilitates the action of the enzyme and allows reasonably speedy and satisfactory separation of the spermatozoa.

The stage of separating the spermatozoa from the resulting mixture in the separation stage (c) herein may be carried out readily using conventional methods, as the main requirement here is that the spermatozoa are protected from the adverse effects of the antagonistic materials in the natural ejaculates but are otherwise virtually unaffected.

A very convenient method is centrifugation, in which case conventional speeds and times in a centrifuge may be used, having due regard for the usual factors such as the viscosity of the mixture and the fragility of the spermatozoa.

The separation may be followed by a washing procedure, to enhance the removal of the undesired materials, and any surplus treatment solution so as to eliminate the enzyme as much as is practicable and avoid any continuing attack on the spermatozoa. Washing is most effectively carried out using an aqueous medium (preferably an isotonic solution), and then centrifuging again, and finally re-suspending the spermatozoa in fresh medium alone. The washing medium should be one which is not unduly hostile towards the spermatozoa or likely to damage them. Most conveniently it may contain an amount of the saccharide (for example at a concentration as in the reagent solution) in one or more of the successive washing/separation stages, as the continuing presence of the saccharide appears to have a beneficial effect. The temperature used for this washing step is best maintained so that it does not adversely affect the spermatozoa. A very suitable temperature is in the range 25 to 30 degrees Centigrade.

The invention also provides preparations containing spermatozoa and having reduced antibody content, produced by the above method. These are preferably used for fertilisation of ova as soon as is practicable, as the viability of the spermatozoa can deteriorate with keeping. However, if desired, the separated concentrates containing the treated spermatozoa may be preserved by conventional methods, for example by storage in sterile low- temperature conditions (with or without appropriate diluents as known in the art) or under cryogenic conditions (in which case the conventional precautions must be taken to prevent damage to the spermatozoa by freezing, for example by adding the conventional cryoprotectants).

When the spermatozoa have been separated, they may be used for fertilisation of ova in a variety of ways. One convenient way is to suspend them in a liquid medium which is pharmaceutically acceptable and not harmful to the spermatozoa (usually a sterile aqueous liquid medium) and preferably isotonic with body fluids. Examples of such liquid media include human tubular-like fluid, Ham's F10 medium, and Tyrode's solution, but any medium which is reasonably compatible with the gametes (i.e. the spermatozoa) may be used if desired.

This re-suspension may be done with a proportion of liquid which gives a convenient number of motile spermatozoa (a "sperm count") for use in fertilisation. A convenient number is usually of the order of $20 \times 16^6$ per millilitre.

The method of the invention and the products so obtained can be used for treatment of semen (sperm) from a variety of different sources, including human or animal sperm, and so may be used in humans and also in veterinary work. It may seem impracticable or uneconomic to use the method of the invention for animals as it will normally be sufficient to deal with a case of infertility in veterinary work by simply using an alternative male and/or female animal. However, sufficient justification may be seen when the male animal donating the sperm is an exceptionally valuable animal (so that it is considered important to secure its progeny in preference to that of an alternative male) as in the cases of breeding from valuable thoroughbred animals and in attempts to breed and preserve rare animal species.

The invention may be put into a form which is most useful for clinical use by making a kit, adapted for the production of a reagent solution as described above. Especially, such a kit comprises separate containers containing respectively:-

(a) a sterile aqueous medium containing such additives as may be required to render it substantially isotonic and/or to act as preservative, either for the solution itself or after use.

(b) a dry mixture of the saccharide and the protease.

Optionally, if the procedure to be followed includes a washing and/or storage step and it is desired to use in the washing and/or storage a liquid which is not the same as the sterile aqueous medium (a), then a third container (c) may be included, containing the washing/storage medium.

In such a kit, of course, there is no necessity to confine it to only single containers of the liquids, and any desired number of these may be included, as desired. Likewise, ancillary equipment, especially a hypodermic syringe may be included for use (as described below) in the mixing and use of the kit to make the reagent solution.

It is preferred that the containers are made of materials suitably chosen with regard for the sensitivity or potential instability of the materials to be kept in them. especially, the container for solution (a) should be made of a material which is resistant to any interaction with the reagent solution to contaminate it; preferably it is made of glass, and especially a borosilicate glass.

The container for dry mixture (b) is adapted so that the dry mixture is maintained under vacuum or in an inert gaseous atmosphere, to assist in preserving the contents. This may be made of glass, but some plastics which are of convenient purity and durability (e.g. polycarbonate, polythene, or the like) may be used.

A most convenient form is that in which one or both of the containers for (a) and/or (b) is provided with a sealed septum through which a hypodermic needle can be inserted. The method for using the kit then comprises inserting a hypodermic needle through the septum of container (a) to withdraw the desired amount of the solution and then inserting the hypodermic needle through the septum of container (b) to inject the solution into it, and then agitating the resulting mixture in container (b) to dissolve the solids and form the desired reagent solution.

The reagent solution can then be warmed to the desired temperature for use.

This form is very practicable because it allows for a user to have the components to hand in appropriate pre-measured amounts and in a sound sterile condition, and to use them with minimum procedural difficulty. The solid enzyme has to be dry to prevent its deterioration on storage, so it is exceptionally convenient to have the two main active components together in the most stable state that can be arranged, ready for use when required.

The preparations of treated spermatozoa obtained by the procedures of this invention may be use for the fertilisation of ova by any of the many known assisted conception or fertilisation methods and procedures. They may be used, for example in "GIFT" (gamete intra-fallopian tube transfer) or IVF (in vitro fertilisation) techniques.

In clinical studies, up to 66% of the ejaculates that show a high total binding with both IgG and IgA antibodies when tested with Immunobeads, become antibody-negative after this treatment or show a higher level of reduction in Immunobead binding. (The name "Immunobeads" is a trademark of BioRad Laboratories.)

The procedure has been shown to have no adverse effect on the fertilising capability of spermatozoa and has been used in assisted fertilisation procedures with ANTI-SPERM antibody-free spermatozoa that have resulted in the birth of normal offspring following assisted conception procedures.

The invention is illustrated but not limited by the following Example in which the parts and percentages are by weight unless otherwise stated.


EXAMPLE 1.

A mixture is prepared by the male donor subject ejaculating directly into a 0.1 M aqueous solution of D-galactose containing chymotrypsin, previously sterilised by passage through an ultrafilter (a 0.22 um membrane). This solution contains 0.05% of chymotrypsin (calculated as the pure enzyme) and 1.8% of the D-galactose. The amount of the solution used is 10 ml., containing 5000 USP units of chymotrypsin (about 500 USP units per ml.) as this -- on the basis of about the sample of semen being about 3ml -- gives an appropriate ratio of the enzyme to semen and also a mixture of conveniently reduced viscosity. The whole mixture is mixed by gentle inversion 5 times.

The mixture is then incubated at 37 degrees C. for 20 minutes, without agitation, to allow the enzyme to act and then is centrifuged at 500g for 5 minutes. The supernatant liquid from this centrifuging is discarded, and the pellet of concentrated spermatozoa is re-suspended in 10.0 ml of a sterile aqueous medium containing 0.1M of D-galactose, by gentle repeated inversion, and the resulting suspension is again centrifuged at 500g for 5 minutes. The supernatant liquid is again discarded.

The resulting pellet of spermatozoa is re-suspended in a further 10.0 ml of plain sterile medium, for example human tubular-like fluid (P. Quinn, J.F. Kerin and G.M. Warnes; Fertil. Steril.; 44 (1985), pages 493-498). The volume is then adjusted if necessary to give a concentration of motile sperm of about 20 x $10^6$ per ml. If this cannot be achieved due to the low count of motile spermatozoa, the pellet may be re-suspended in 0.5 ml of the medium.

This preparation of spermatozoa is suitable for use in GIFT (gamete intra-fallopian tube transfer) or IVF (in vitro fertilisation) techniques.

In clinical studies, up to 66% of the ejaculates that show a high total binding with both IgG and IgA antibodies when tested with Immunobeads, become antibody-negative after this treatment or show a higher level of reduction in Immunobead binding. (The name "Immunobeads" is a trademark of BioRad Laboratories.)

The procedure has been shown to have no adverse effect on the fertilising capability of spermatozoa and has been used in assisted fertilisation procedures with anti-sperm antibody-free spermatozoa that have resulted in the birth of normal offspring following assisted conception procedures.

The results are summarised below in Table 1.

TABLE 1.

| Patient | Ejaculate Volume (ml) | Initial Total Sperm Concentration (x $10^6$/ml) | Initial Mobile Sperm Concentration (x $10^6$/ml) | Final Immunobead Binding (Initially 100%) |
|---------|------------------------|--------------------------------------------------|---------------------------------------------------|--------------------------------------------|
| 1 | 0.8 | 50 | 18 | 100% IgG<br>100% IgA |
| 2 | 3.6 | 92 | 9.2 | 26% IgG<br>65% IgA |
| 3 | 1.0 | 61 | 47 | 100% IgG<br>100% IgA |
| 4 | 2.5 | 9.1 | 1.7 | 0% IgG<br>0% IgA |
| 5 | 1.0 | 246 | 216 | 100% IgG<br>100% IgA |
| 6 | 8.7 | 38 | 3.5 | 41% IgG<br>50% IgA |
| 7 | 2.8 | 7.7 | 1.8 | 0% IgG<br>0% IgA |
| 8 | 4.5 | 114 | 5 | 14% IgG<br>40% IgA |
| 9 | 3.2 | 52 | 24.4 | 0% IgG<br>0% IgA |

These results show that in three of the nine cases treated, all of the antibody has been removed and in a further three cases about 50% has been removed. In these six cases sperm motility in both artificial medium and cervical mucus has now been established, having previously been absent.

EXAMPLE 2.

Displacement of anti-sperm antibodies from spermatozoa using D-galactose and chymotrypsin.

For the baseline estimation of antibody levels, patients ejaculated directly into human tubular-like fluid (HTF, Quinn et al., 1985), those who scored 3+ in the mixed agglutination reaction for both IgG and IgA antibodies or were 100% positive for IgG and IgA Immunobead binding were selected for the following test.

The test condition involved ejaculation into 10 ml of HTF medium containing 0.1M D-galactose and 5000 USP units of chymotrypsin. The mixture was incubated for 20 minutes at 37 degrees Centigrade, the spermatozoa were separated by centrifugation at 500 g for 10 minutes, washed in HTF medium containing 0. 1M D-galactose, re-centrifuged, and suspended at a suitable concentration of spermatozoa in HTF medium before re-testing for Immunobead binding or cervical mucus penetration.

EXAMPLE 3.

A group of sixteen infertile couples were chosen who had suffered from long-standing primary infertility in which the only detectable cause was a high titre of antisperm antibodies in the husband's semen.

By treatment of the husband's semen with a solution of chymotrypsin and D-galactose, in the manner described in Example 1, the treated spermatozoa were used to fertilise the wife's ova, and five pregnancies resulted following a single treatment using this procedure.

The methods of fertilisation used were:

(a) Seven In Vitro Fertilisations (IVF), which yielded two pregnancies.

(b) Three Gamete Intrafallopian Tube Transfers (GIFT), which yielded two pregnancies.

(c) Six FREDI procedures (a combination of IVF and intra-uterine insemination), which yielded one pregnancy.

**Claims**

6

1. Method for treating semen and/or spermatozoa which comprises:-
(a) mixing the semen with a reagent solution containing a protease and a saccharide,
(b) incubating the mixture under conditions at which the protease is active, and then
(c) separating the spermatozoa from the resulting mixture.

2. Method as claimed in Claim 1 wherein the saccharide is derived from galactose, and is preferably D-galactose.

3. Method as claimed in Claim 1 or Claim 2 wherein the protease (proteolytic enzyme) is free from DNAase (i.e. any enzyme which degrades DNA) and is active at substantially neutral pH, and is preferably a serine protease, for example chymotrypsin.

4. Method for the treatment of semen/spermatozoa substantially as described.

5. A reagent solution, useful for carrying out a method as claimed in any of Claims 1 to 4, which comprises a protease and a saccharide and is preferably made as a solution in an aqueous medium, preferably substantially isotonic with body fluids, optionally containing one or more additives to assist in the preservation of the reagent itself or the spermatozoa.

6. A reagent solution substantially as described.

7. A kit for production of the reagent solution as claimed in Claim 5 or Claim 6, which comprises separate containers containing respectively:-
(a) a sterile aqueous medium containing such additives as may be required to render it substantially isotonic and/or to act as preservative, either for the solution itself or after use.
(b) a dry mixture of the saccharide and the protease. and optionally
(c) a washing and/or storage medium.

8. A kit for the treatment of semen/spermatozoa substantially as described.

9. Method for the use of a kit as claimed in Claim 7 or 8, which comprises inserting a hypodermic needle through the septum of container (a) to withdraw the desired amount of the solution and then inserting the hypodermic needle through the septum of container (b) to inject the solution into it, and then agitating the resulting mixture in container (b) to dissolve the solids and form the desired reagent solution.

10. A preparation containing spermatozoa and having reduced antibody content, produced by a method as claimed in any of Claims 1 to 4 or by use of a reagent solution as claimed in any of Claims 5 to 6 or by use of a kit as claimed in any of Claims 7 to 8, for example by a method as claimed in Claim 9.

European . Patent Office

**PARTIAL EUROPEAN SEARCH REPORT**
which under Rule 45 of the European Patent Convention shall be considered, for the purposes of subsequent proceedings, as the European search report

Application number

EP 89310917.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.⁵) |
|---|---|---|---|
| X | WPI 2953600, Acc no. 82-06589, J/49; & SU A - 904 691 (BELO LIVESTOCK RES.) 15-02-1982 * the whole document * | 5 | A61K35/52 //A61K37/54 |
| A | MEDIZINISCHE WELT vol. 26, no. 33, 1975, pages 1450-1453, Stuttgart, DE; W.-B. SCHILL: "Die Bedeutung der Proteasen des männlichen Genitaltraktes für die Fortpflanzung" * page 1451, column 1, paragraph 2 * | 5 | |
| A | US - A - 3 718 740 (H.D. HAFS et al.) * the whole document * | 10 | |
| A | WO - A - 8 401 265 (GENETIC ENGINEERING INC) * page 5, lines 1-30 * | 5,7,10 | |

TECHNICAL FIELDS SEARCHED (Int. Cl.⁵)

A61K
A61D

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 5-10
Claims searched incompletely:
Claims not searched: 1-4: Method for treatment of human or
Reason for the limitation of the search: animal body by therapy (art. 52(4))

| Place of search | Date of completion of the search | Examiner |
|---|---|---|
| BERLIN | 15-01-1990 | A.J. DE KOK |